# EUROPÄISCHE PATENTANMELDUNG

(11) **EP 0 835 875 A1**
(43) Veröffentlichungstag der Anmeldung: **15.04.1998**
(21) Anmeldenummer: 97117091.5
(22) Anmeldetag: 02.10.1997
(51) Int. Cl.: C07D 487/04, C08K 5/34

(54) **Stabilisatoren auf Basis von 1-Aza-2,2,6,6-tetramethylbicyclo(3.1.0)hexan**

(30) Priorität: 11.10.1996 DE 19641905
(71) Anmelder: Clariant GmbH, 65929 Frankfurt am Main (DE)
(72) Erfinder: Stährfeldt, Thomas, Dr., 86356 Neusäss (DE); Wiedemann, Josef, Dr., 86441 Zusmarshausen (DE); Zäh, Matthias, Dr., 86368 Gersthofen (DE)

(57) **Zusammenfassung**

Die vorliegende Erfindung bezieht sich auf neue Stabilisatoren der allgemeinen Formel (I) 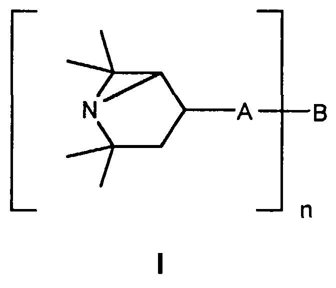 worin die Substituenten die in der Beschreibung definierte Bedeutung besitzen.

Diese neuen Stabilisatoren sind sehr gut geeignet zum Stabilisieren von organischem Material wie Kunststoffen, Ölen, Anstrichmitteln und Lacken gegen den Abbau durch Licht, Strahlung, Wärme und Sauerstoff.

## Beschreibung

Es ist bekannt, daß organische Materialien durch Licht, Strahlung, Wärme oder Sauerstoff geschädigt werden. Es gibt bereits viele Druckschriften, die Verbindungen zur Stabilisierung von organischem Material beschreiben. Im Zusammenhang mit Polyolefinen handelt es sich meist um Verbindungen auf der Basis von sterisch gehinderten Phenolen, organischen Phosphiten, α-Hydroxybenzophenon, Hydroxyphenylbenzotriazolen, Nickelkomplexen oder 2,2,6,6-Tetraalkylpiperidin (vgl. R. Gächter, H. Müller, Plastics Additives Handbook, 3rd Ed., Hanser Verlag, München 1990).
Die genannten Verbindungsklassen weisen oft spezifische Nachteile auf, die neben der erwünschten stabilisierenden Wirkung auftreten. Besonders bezüglich Farbverhalten, Wechselwirkung mit Pigmenten, Verträglichkeit der verschiedenen Stabilisatoren untereinander und mit dem zu stabilisierenden Material, Resistenz gegen Chemikalien und Wasser (Hydrolyseempfindlichkeit), Lagerstabilität, Migrationsverhalten und Verbesserung der Stabilisierung gegen die schädigenden Einflüsse von Wärme und Licht im Langzeitgebrauch besteht ein großer Bedarf nach neuen Stabilisatorklassen.

Aufgabe der vorliegenden Erfindung war es somit, neue Stabilisatoren zur Verfügung zu stellen, die die beschriebenen Nachteile nicht aufweisen.

Es wurde überraschenderweise gefunden, daß geeignete Derivate des 1-Aza-2,2,6,6-tetramethylbicyclo[3.1.0]hexan (**I**) in der Lage sind, organische Materialien gegen den schädigenden Einfluß von Licht, Strahlung, Wärme und Sauerstoff ausgezeichnet zu stabilisieren.

Gegenstand der Erfindung sind somit neue Stabilisatoren, die mit der allgemeinen Formel (**I**) beschrieben werden können: 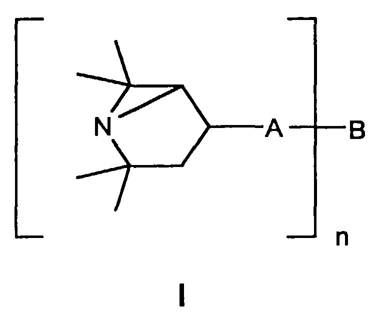
**n** ist 1 oder 2,
**A** bedeutet:
   -O-C(O)-, -O-C(O)-N(H)-, -N(R¹)-C(O)-, -N(R¹)-C(O)-N(H)-, oder eine direkte Bindung, vorzugsweise -O-C(O)- oder -O-C(O)-N(H)-,
**B** bedeutet, falls **A** eine direkte Bindung darstellt:
   - für **n** = 1:: -N(R¹R²) oder-O-R¹ ,
   - für **n** = 2:: -N(R¹)- ,
**B** bedeutet, falls **A** keine direkte Bindung darstellt:
   - für **n** = 1:: C₂-C₁₈-Alkyl, vorzugsweise C₈-C₁₆-Alkyl, C₃-C₁₀-Cycloalkyl, vorzugsweise C₄-C₈-Cycloalkyl, mit -CN, -NO₂, Amin oder Halogen substituiertes C₆-C₁₈-Aryl oder C₇-C₁₈-Arylalkyl, vorzugsweise C₆-C₁₀-Aryl oder C₇-C₁₀-Arylalkyl, oder einen heteroaromatischen Rest mit 5-15 C-Atomen, vorzugsweise 6-10 C-Atomen,
   - für **n** = 2:: C₂-C₁₈-Alkylen, vorzugsweise C₄-C₈-Alkylen, mit -CN, -NO₂, Amin oder Halogen substituiertes C₆-C₁₈-Arylen oder C₇-C₁₈-Arylalkylen, vorzugsweise C₆-C₁₀-Arylen oder C₇-C₁₀-Arylalkylen, oder einen heteroaromatischen Rest mit 5-15 C-Atomen, vorzugsweise 6-10 C-Atomen.
**R**^{**1**} bedeutet:
   H, ein substituiertes C₁-C₁₈-Alkyl oder -Alkylen, vorzugsweise ein mit einem Derivat des Triazin oder mit einem Amin substituiertes C₂-C₈-Alkyl oder -Alkylen oder ein mit einem Derivat des 1-Aza-2,2,6,6-tetramethylbicyclo[3.1.0]hexan substituiertes C₁-C₁₈-Alkyl, C₃-C₁₀-Cycloalkyl, mit -CN, -NO₂, Amin oder Halogen substituiertes C₆-C₁₈-Aryl oder C₇-C₁₈-Arylalkyl, vorzugsweise C₆₋C₁₀₋Aryl oder C₇-C₁₀-Arylalkyl, oder einen heteroaromatischen Rest mit 5-15 C-Atomen, vorzugsweise 6-10 C-Atomen.
**R**^{**2**} bedeutet:
   H, ein substituiertes C₁-C₁₈-Alkyl, vorzugsweise ein mit einem Derivat des Triazin oder mit einem Amin substituiertes C₂-C₈-Alkyl, C₃-C₁₀-Cycloalkyl, mit -CN, -NO₂, Amin oder Halogen substituiertes C₆-C₁₈-Aryl oder C₇-C₁₈-Arylalkyl, vorzugsweise C₆-C₁₀-Aryl oder C₇-C₁₀-Arylalkyl, oder einen heteroaromatischen Rest mit 5-15 C-Atomen, vorzugsweise 6-10 C-Atomen, oder ein Derivat des Triazins, vorzugsweise ein Derivat des Triazins der Formel T₁ oder T₂.

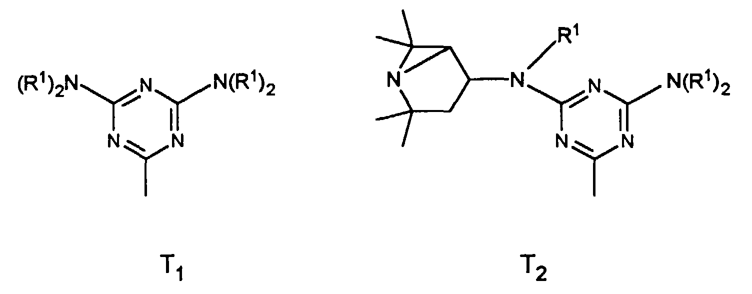

Die genannten heteroaromatischen Reste enthalten bis zu 3, vorzugsweise 1 oder 2 Heteroatome. Als Heteroatome werden O, S und N, vorzugsweise N verwendet.

Die vorliegende Erfindung bezieht sich ferner auf die Art der Herstellung dieser neuen Verbindungen und weiterhin auf die Verwendung der neuen Verbindungen als Stabilisatoren von organischem Material gegen den schädigenden Einfluß von Licht, Strahlung, Wärme und Sauerstoff.

Verbindung II wurde, ausgehend von 2,2,6,6-Tetramethyl-4-oxo-piperidin, analog den Angaben aus der Literatur in einer zweistufigen Synthese und in guter Ausbeute hergestellt (L. A. Krinitskaya, Izv. Akad. Nauk SSSR, Ser. Khim. 7 (1987) 1685; CAS 108:150284h).

### Bild 1: Allgemeines Syntheseschema der erfindungsgemäßen Verbindungen, ausgehend von 1-Aza-4-oxo-2,2,6,6-tetramethylbicyclo[3.1.0]hexan (II).

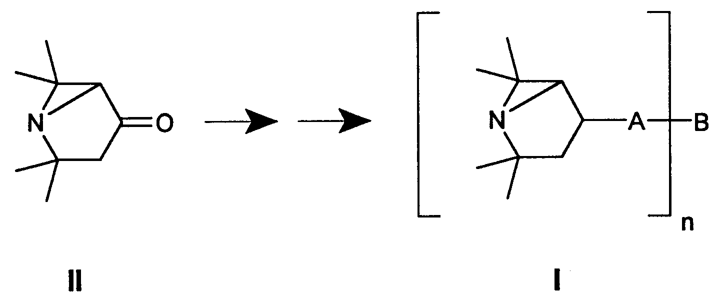

Es wurde gefunden, daß die Ketofunktion in II für weitere Umsetzungen zugänglich ist. Sie kann z. B. zum entsprechenden Alkohol (III) reduziert oder zweitens mit Aminen zu entsprechenden Iminen (IV) umgesetzt werden. Das Imin kann weiter zum entsprechenden Amin (V) reduziert werden (Bild 2).

### Bild 2: Allgemeines Syntheseschema des erfindungsgemäßen Verfahrens zur Herstellung der Vorstufen III und V. R¹ hat die oben angegebene Bedeutung.

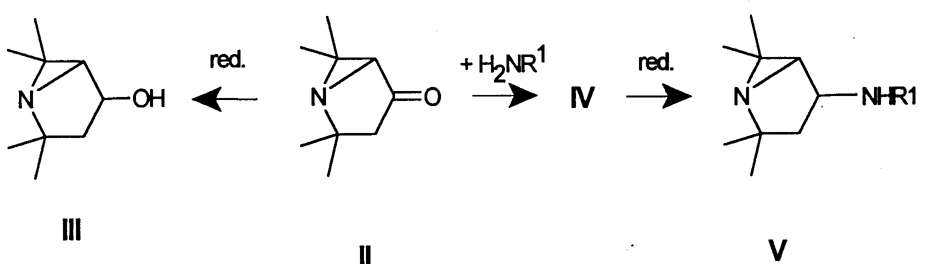

Beim Betrachten der Produkte III und V fällt auf, daß es sich jeweils um Diastereomerenpaare handelt. Die Diastereomeren der Verbindung III wurden voneinander getrennt. Die Folgereaktionen von III wurden mit einem reinen Diastereomer und mit dem Gemisch der beiden Diastereomeren, die Folgereaktionen von V nur mit dem Gemisch der beiden Diastereomeren durchgeführt.

Die Verbindungen III, IV und V sind, im Gegensatz zur vorbeschriebenen Vorstufe II, Gegenstand der Erfindung. Bei diesen Verbindungen handelt es sich um Alkohole (III) oder um primäre bzw. sekundäre Amine (V) (abhängig davon, ob es sich bei R¹ um ein H-Atom oder um einen der übrigen weiter oben näher ausgeführten Reste handelt). Diese erfindungsgemäßen Verbindungen III und V können mit einem geeigneten Reagenz zur ebenfalls erfindungsgemäßen Verbindung I umgesetzt werden.

Die Umsetzung von 2,2,6,6-Tetramethyl-4-oxo-piperidin II mit einem Amin zum entsprechenden Imin des Typs IV kann beispielsweise in einem aprotischen, organischen Lösemittel, vorzugsweise einem Kohlenwasserstoff oder in einem Ether, insbesondere in Methyl-tert.butylether durchgeführt werden. Das Amin kann stöchiometrisch oder im Überschuß eingesetzt werden. Eine weitere Möglichkeit besteht darin, das Amin im Überschuß als Lösemittel zu verwenden. Die Umsetzung kann auch durch eine saure Verbindung katalysiert werden, wie z. B. durch organische Säuren, insbesonders durch p-Toluolsulfonsäure. Als Reaktionstemperatur wird eine Temperatur gewählt, die höher ist als 20 °C, besonders gut eignet sich die Temperatur des Siedepunktes des verwendeten Lösungsmittels.

Die Reduktion eines Imins des Typs IV zum entsprechenden Amin Typs V bzw. die Reduktion von 2,2,6,6-Tetramethyl-4-oxo-piperidin II zum entsprechenden Alkohol 2,2,6,6-Tetramethyl-4-hydroxy-piperidin III kann in einem protischen oder aprotischen, organischen Lösemittel erfolgen, vorzugsweise in Tetrahydrofuran oder einem Alkohol insbesonders in Methanol oder Ethanol oder in Gemischen dieser geeigneten Lösungsmittel. Als Reduktionsmittel kann ein komplexes Hydrid, insbesonders NaBH₄ verwendet werden, welches vorzugsweise stöchiometrisch oder in leichtem Überschuß bezogen auf das Imin bzw. das Keton, eingesetzt werden. Zur Aufarbeitung kann das Lösungsmittel destillativ entfernt, der Rückstand mit einem polaren Lösungsmittel versetzt und das Produkt mit einem organischen Lösungsmittel extrahiert werden. Als polares Lösungsmittel ist vorzugsweise Wasser geeignet, insbesonders jedoch eine wäßrige Lösung von einem anorganischen Salz, vorzugsweise NaCl. Als organisches Lösungsmittel für die Extraktion eignet sich vorzugsweise ein mit Wasser nicht mischbares, organisches Lösungsmittel, insbesonders ein Ether wie z. B. Methyl-tert.butylether.

Die Umsetzung des Alkohols 2,2,6,6-Tetramethyl-4-hydroxy-piperidin III oder des Amins des Typs V zu den erfindungsgemäßen Verbindungen I erfolgt in einem aprotischen, organischen Lösemittel, vorzugsweise einem Kohlenwasserstoff insbesondere aromatischen Kohlenwasserstoffen wie beispielsweise Toluol, Xylol oder in Gemischen hieraus oder in Tetrahydrofuran. Eine weitere Möglichkeit besteht darin, eine der Reaktionskomponenten im Überschuß als Lösemittel zu verwenden. Bei diesen geeigneten Reaktionskomponenten handelt es sich um Verbindungen, die bekannterweise unter geeigneten Bedingungen mit Alkoholen, primären oder sekundären Aminen reagieren. Bei solchen Verbindungen handelt es sich beispielsweise um gegebenenfalls weiter substituierte mono-/ bzw. difunktionelle Isocyanate, Ester, Säurehalogenide, Säureanhydride, Alkylhalogenide, halogensubstituierte Triazine.

Die erfindungsgemäßen Verbindungen eignen sich in hervorragender Weise zum Stabilisieren von organischem Material gegen die Einwirkung von Licht, Strahlung, Sauerstoff und Wärme. Sie werden dem zu stabilisierenden organischen Material in einer Konzentration von 0,001 bis 5 Gew.-%, vorzugsweise von 0,02 bis 1,0 Gew.-%, bezogen auf das organische Material, vor, während oder nach seiner Herstellung zugesetzt.

Unter organischem Material sind beispielsweise Vorprodukte für Kunststoffe, Lacke, Anstrichmittel und Öle, insbesondere jedoch Kunststoffe, Lacke, Anstrichmittel und Öle selbst, zu verstehen.

Gegenstand der vorliegenden Erfindung sind außerdem gegen die Einwirkung von Licht, Strahlung, Sauerstoff und Wärme stabilisierte organische Materialien, insbesondere Kunststoffe, Lacke, Anstrichmittel und Öle, welche die in dieser Anmeldung beanspruchte(n) Verbindung(en) in den oben angegebenen Konzentrationen enthalten. Zu diesen organischen Materialien gehören beispielsweise Stoffe, wie sie in DE P 4423055.9, Seite 13 - 18, beschrieben sind.

Das durch die erfindungsgemäßen Verbindungen stabilisierte organische Material kann gegebenenfalls noch weitere Additive enthalten, beispielsweise Antioxidantien, Lichtstabilisierungsmittel, Metalldesaktivatoren, antistatische Mittel, flammhemmende Mittel, Pigmente und Füllstoffe.
Antioxidantien und Lichtstabilisatoren, die neben den erfindungsgemäßen Verbindungen zugesetzt werden, sind beispielsweise Verbindungen auf der Basis sterisch gehinderter Amine; Antioxidantien sind z. B. sterisch gehinderte Phenole oder Schwefel bzw. Phosphor enthaltende Costabilisatoren.
Als geignete zusätzliche Additive kommen beispielsweise Verbindungen, wie in DE P 4423055.9, Seite 18-29 dargestellt, in Betracht. Als weitere geeignete Additive kommen zusätzlich in Betracht 2,2',2''-Nitrilo[triethyl-tris(3,3',5,5'-tetra-tert.butyl-1,1'-biphenyl-2,2'-diyl)phosphit], Bis[2-methyl-4,6-bis(1,1-dimethylethyl)phenol]phosphorigsäureethylester, sekundäre Hydroxylamine wie z. B. Distearylhydroxylamin oder Dilaurylstearylamin, Zeolithe wie z. B. DHT 4A, Oxide und Hydroxide von Aluminium, Zink, Alkali- und Erdalkalimetalle, wobei für einzelne Anwendungen besonders feinkörniges Material speziell geeignet ist, Al-, Ca-, Mg-, Zn-Stearat, wobei für einzelne Anwendungen besonders feinkörniges Material speziell geeignet ist, Kondensationsprodukt aus N,N'-Bis[(4,6-di(4-n-butylamino-2,2,6,6-tetramethylpiperid-4-yl)1,3,5-triazin-2-yl]-3-aminopropyl-ethylen-1,2-diamin und 2,4-Dichlor-6-(4-n-butylamino-2,2,6,6-tetramethyl-piperid-4-yl)1,3,5-triazin, 1,3,-2,4-Di(benziliden)-D-sorbitol, 1,3-2,4-Di-(4-tolyliden)-D-sorbitol, 1,3-2,4-Di(4-ethylbenziliden)-D-sorbitol.

Mischungsbestandteil können auch Hydrotalcite sein, die beschrieben werden können nach der allgemeinen Formel

[(M²⁺)₁₋ₓ (M³⁺)ₓ (OH)₂ (Aⁿ⁻)_{x/n}yH₂O],

wobei
- (M²⁺): bedeutet Mg, Ca, Sr, Ba, Zn, Pb, Sn, Ni,
- (M³⁺): bedeutet Al, B, Bi,
- Aⁿ: bedeutet ein Anion der Wertigkeit n,
- n: bedeutet eine ganze Zahl von 1 bis 4,
- x: bedeutet einen Wert zwischen 0 und 0,5,
- y: bedeutet einen Wert zwischen 0 und 2,
- A: bedeutet OH⁻, Cl⁻, Br⁻, I⁻, ClO⁴⁻, CH₃COO⁻, C₆H₅COO⁻, CO₃²⁻, SO₄²⁻, (OOC-COO)²⁻, (CHOHCOO)₂²⁻, (CHOH)₄CH₂OHCOO⁻, C₂H₄(COO)₂²⁻, (CH₂COO)₂²⁻, CH₃CHOHCOO⁻, SiO₃²⁻, SiO₄⁴⁻, Fe(CN)₆³⁻, Fe(CN)₆⁴⁻, BO₃³⁻, PO₃³⁻, HPO₄²⁻.
Bevorzugt eingesetzt werden Hydrotalcite, in welchen (M²⁺) für (Ca²⁺), (Mg²⁺) oder eine Mischung aus (Mg²⁺) und (Zn²⁺); (Aⁿ⁻) für CO₃²⁻, BO₃³⁻, PO₃³⁻ stehen. x hat einen Wert von 0 bis 0,5 und y hat einen Wert von 0 bis 2.

Weiterhin können auch Hydrotalcite, die mit der Formel

[(M²⁺)ₓ (Al³⁺)₂ (OH)_{2x+6nz} (Aⁿ⁻)₂ yH₂O]

beschrieben werden können, eingesetzt werden.
Hier steht (M²⁺) für Mg^{2+r} oder Zn²⁺, bevorzugterweise jedoch Mg²⁺.
(Aⁿ⁻) steht für ein Anion, besonders aus der Gruppe CO₃²⁻, (OOC-COO)²⁻, OH⁻ und S²⁻, wobei n die Wertigkeit des Ions beschreibt.
y steht für eine positive Zahl zwischen 0 und 5, besonders zwischen 0,5 und 5;
x und z haben positive Werte, die bei x zwischen 2 und 6 liegen und bei z kleiner als 2 sein sollten. Besonders bevorzugt sind die Hydrotalcite der folgenden Formeln anzusehen:

Al₂O₃ · 6MgO · CO₂ · 12H₂O , Mg_{4.5}Al₂(OH)₁₃ · CO₃ · 3.5H₂O , 4MgO · Al₂O₃ · CO₂ · 9H₂O , 4MgO · Al₂O₃ · CO₂ · 6H₂O , ZnO · 3MgO · Al₂O₃ · CO₂ · 8-9H₂O , ZnO · 3MgO · Al₂O₃ · CO₂ · 5-6H₂O , Mg_{4.5}Al₂(OH)₁₃ · CO₃ .

Hydrotalcite werden im Polymer bevorzugt in einer Konzentration von 0,01 bis 5 Gew.%, besonders von 0,2 bis 3 Gew.%, bezogen auf die gesamte Polymerzubereitung, eingesetzt.

Die Additive werden nach allgemein üblichen Methoden in die organischen Polymere eingearbeitet. Die Einarbeitung kann beispielsweise durch Einmischen oder Aufbringen der Verbindungen und gegebenenfalls weiterer Additive in oder auf das Polymere unmittelbar nach der Polymerisation oder in die Schmelze vor oder während der Formgebung erfolgen. Auch durch Aufbringen der gelösten oder dispergierten Verbindungen auf das Polymere direkt oder durch Einmischen in eine Lösung, Suspension oder Emulsion des Polymeren, gegebenenfalls unter nachträglicher Verdunstung des Lösemittels, kann die Einarbeitung erfolgen. Die Verbindungen sind auch wirksam, wenn sie in ein bereits granuliertes Polymer nachträglich in einem gesonderten Verarbeitungsschritt eingebracht werden. Die erfindungsgemäße(n) Verbindung(en) der Formel (I) kann auch in Form eines Masterbatches, der diese Verbindung(en) beispielsweise in einer Konzentration von 1 bis 75, vorzugsweise 2,5 bis 30 Gew.-% enthält, den zu stabilisierenden Polymeren zugesetzt werden.

### Beispiele

Die nachfolgenden Beispiele sollen die Erfindung näher erläutern. Alle Verbindungen konnten anhand ihrer ¹H- bzw. ¹³C-NMR-Spektren eindeutig identifiziert werden. Die Synthesen wurden mit kommerziell erhältlichen Edukten durchgeführt.

### 1. Herstellung der nicht erfindungsgemäßen Edukte

### 1.1. Herstellung von N-Chlor-2,2,6,6-tetramethyl-4-oxo-piperidin (M = 189,7 g/mol)

500,0 g (2,10 mol) Dichlorisocyanursäure-Na-Salz-Dihydrat werden in 1800 ml Wasser gelöst und bei 45 °C unter Rühren langsam zu einer Lösung von 296,0 g (1,909 mol) 2,2,6,6-Tetramethyl-4-oxo-piperidin in 1000 ml Wasser gegeben (10 min.). Die dünnflüssige, leicht gelbe Suspension wird 180 min. bei 30 °C nachgerührt. Nach Zugabe von 760 ml Toluol wird bei 25 °C weitere 90 min. intensiv gerührt. Der Feststoff wird abfiltriert und mit 300 ml Toluol gewaschen. Nach Abtrennen der organischen Phase wird die wäßrige Phase dreimal mit je 200 ml Toluol ausgeschüttelt. Die organischen Phasen werden vereinigt, mit Natriumsulfat getrocknet, von diesem abfiltriert und im Vakuum vom Lösungsmittel befreit. Die Rohausbeute (359,0 g; 1,89 mol; 99%; oranges Öl) wird bei einem verminderten Druck destilliert. Bei einer Abgangstemperatur von 112 °C/0.01 mbar werden 333,0 g (1,76 mol, 92 %) Produkt isoliert (hellgelbes Öl).

### 1.2. Herstellung von 1-Aza-4-oxo-2,2,6,6-tetramethylbicyclo[3.1.0]hexan (II) (M = 153,2 g/mol)

450 g einer 30 %igen methanolischen Natriummethanolat-Lösung (135,0 g NaOMe, 2,50 mol) werden unter Rühren in eine Lösung von 379,4 g (2,0 mol) N-Chlor-2,2,6,6-Tetramethyl-4-oxo-piperidin in 980 ml Methanol bei 66 °C im Laufe von 80 min. zugegeben. Nach dem Zutropfende wird die Lösung 150 min. bei 66 °C gerührt. Der Feststoff wird bei 20 °C filtriert und mit 100 ml Methanol gewaschen. Das Filtrat wird bei 50 °C und 20 mbar vom Lösungsmittel befreit. Der Rückstand wird mit 300 ml 10 %iger NaCl-Lösung versetzt und dreimal mit je 200 ml Methyl-tert.butylether ausgeschüttet. Die vereinigten organischen Phasen werden mit Natriumsulfat getrocknet, von diesem abfiltriert und bei 12 mbar vom Lösungsmittel befreit. Das verbleibende orange Öl wird bei einem verminderten Druck destilliert. Bei einer Abgangstemperatur von 82 °C/15 mbar werden 276,0 g (1,80 mol, 90 %) Produkt isoliert (goldgelbes Öl).

### 2. Herstellung der erfindungsgemäßen Produkte III, D und E

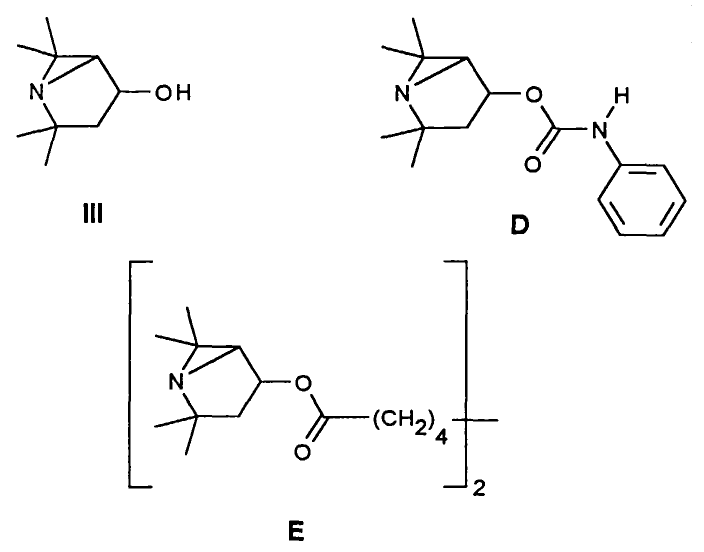

### 2.1. Herstellung von 1-Aza-4-hydroxy-2,2,6,6-tetramethylbicyclo[3.1.0]hexan (III) (M = 155,27 g/mol)

In eine Lösung von 306,4 g (2.00 mol) 1-Aza-4-oxo-2,2,6,6-tetramethylbicyclo[3.1.0]hexan (II) in 1000 ml Ethanol werden 60,4g (1,60 mol) NaBH₄ unter Kühlung bei 20 °C portionsweise zugegeben (20 min.). Die Mischung wird 120 min. bei 20 °C und weitere 120 min. bei 50 °C gerührt, nach der Gasentwicklung entsteht eine milchig weiße Mischung. Bei 12 mbar und 60 °C wird das Lösungsmittel entfernt. Der Rückstand wird mit 300 ml 10 %iger NaCl-Lösung versetzt und mit je 200 ml Methyl-tert.butylether dreimal ausgeschüttelt. Die vereinigten organischen Phasen werden mit Natriumsulfat getrocknet, von diesem abfiltriert und bei 12 mbar / 75 °C eingeengt. Aus der Rohausbeute von 270,3 g (1,74 mol; 87%) kristallisiert nach 18 Stunden bei 20 °C eines der Diastereomeren in Form von farblosen Kristallen aus. Das Isomerengemisch wird mit 50 g 3-Pentanon verrührt, der unlösliche Anteil wird abfiltriert (132,6g; 0,854 mol; 43 %; Fp.= 114-116 °C; Diastereomeren-Verhältnis nach GC: 91:9). Das feste Produkt wird aus 3-Pentanon umkristallisiert. Ausbeute: 114,6 g (0,734 mol); Fp. = 120 - 121 °C; ¹³C{¹H}-NMR (CDCl₃): δ = 18.6, 25.3, 29.5, 33.7, 40.8, 46.6, 53.4, 62.6, 71.9 ppm.

Das Filtrat wird bei 12 mbar / 50 °C vom Lösungsmittel befreit. Die zweite Produktfraktion wird als hellgelbes, trübes Öl isoliert (112,2 g; 0,721 mol; 36 %; Diastereomeren-Verhältnis nach GC: 46:54). Beim als Öl anfallenden Anteil handelt es sind nach ¹H- und ¹³C-NMR um ein Gemisch aus beiden Diastereomeren.

### 2.2. Herstellung von Verbindung D (M = 274,40 g/mol)

Bei 25 °C werden zu einer Lösung von 31,1 g (0,2 mol) 1-Aza-4-hydroxy-2,2,6,6-tetramethylbicyclo[3.1.0]hexan (III) in 100 ml Toluol unter Rühren 26,2 g (0,22 mol) Phenylisocyanat langsam zugegeben; dabei erwärmt sich das Gemisch auf 50 °C. Nach 30 min. Rühren bei 45 °C ergibt sich eine starke Trübung und im Laufe von weiteren 200 min. Rühren bei 25 °C fällt ein dicker Niederschlag aus. Der Niederschlag wird über eine Glassinterfritte abgesaugt, mit 50 ml Toluol und 100 ml Hexan gewaschen und bei 100 °C im Vakuum getrocknet. Ausbeute: 45,1 g (0,164 mol; 82%), farblose Kristalle, Fp.: 146 °C.

### 2.2. Herstellung von Verbindung E (M = 448,72 g/mol)

Ein Gemisch aus 23,0 g (0,10 mol) Sebacinsäuredimethylester, 35,7 g (0,23 mol) 1-Aza-4-hydroxy-2,2,6,6-tetramethylbicyclo[3.1.0]hexan (III) (es wird der kristalline Anteil mit einem Diastereomerenverhältnis von 91:9 verwendet) und 0,15 g Lithiumamid in 250 ml Heptan wird 6 h bei 98 °C gerührt, wobei das langsam entstehende Methanol kontinuierlich aus dem Gleichgewicht entfernt wird. Das Gemisch wird bei 95 °C mit Wasser ausgeschüttelt. Die organische Phase wird bei 95 °C von der wäßrigen Phase getrennt, mit Natriumsulfat getrocknet, von diesem abfiltriert und bei 12 mbar / 50 °C vom Lösungsmittel befreit. Ausbeute: 44,1 g (0,098 mol; 98%), farbloses Öl, Kp. 287 °C, im ¹³C-NMR rein (Carbonylsignal in CDCl₃ bei δ=172.74 ppm).
Der Versuch wurde analog mit dem öligen Produkt III (Diastereomerenverhältnis von 46:54) durchgeführt. Ausbeute: 86%, farbloses Öl.

### 3. Herstellung der erfindungsgemäßen Verbindungen des Typs IV

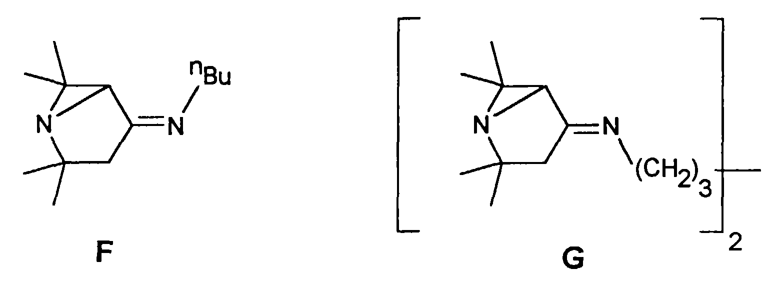

### 3.1. Herstellung von Verbindung F (M = 208,39 g/mol)

Eine Lösung von 80,0 g (0,523 mol) 1-Aza-4-oxo-2,2,6,6-tetramethylbicyclo[3.1.0]hexan (II), 38,3 g (0,523 mol) n-Butylamin, 85,2 g (0,575 mol) Orthoameisensäuretriethylester, 0,3 g p-Toluolsulfonsäure in 260 ml Methyl-tert.-butylether wird 13 h bei 62 °C gerührt. Die goldgelbe, klare Lösung wird bei einem Druck von 50 mbar und 40 °C vom Lösungsmittel befreit. Das flüssige Rohprodukt wird im Vakuum destilliert; bei einer Abgangstemperatur von 118 °C/15 mbar wird das Produkt als goldgelbes, klares Öl isoliert (89,0 g; 0,429 mol; 82 %).

### 3.2. Herstellung von Verbindung G (M = 386,70 g/mol)

Eine Mischung von 76,6 g (0,5 mol) 1-Aza-4-oxo-2,2,6,6-tetramethylbicyclo[3.1.0]hexan (II), 29,1 g (0,25 mol) Hexamethylendiamin, 92,0 g (0,612 mol) Orthoameisensäuretriethylester, 0,4 g p-Toluolsulfonsäure in 250 ml Methyl-tert.butylether wird 10 h bei 66 °C gerührt. Die goldgelbe, klare Lösung wird bei 50 °C und 25 mbar eingeengt, das verbleibende Öl wird bei vermindertem Druck destilliert. Das Produkt wird bei einer Abgangstemperatur von 174 °C (0,008 mbar) isoliert. Ausbeute: 69,3 g (0,179 mol; 72%), oranges, klares Öl.

### 4. Herstellung der Verbindungen des Typs V

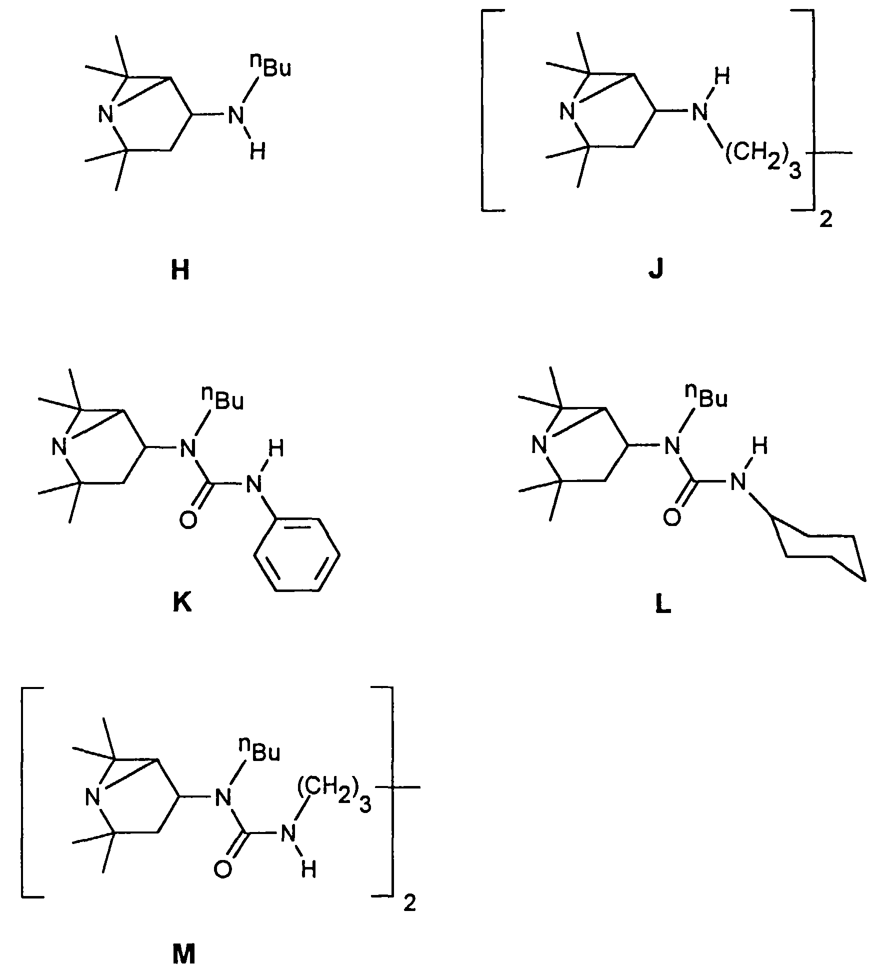

### 4.1. Herstellung von 1-Aza-4-(n-butylamino)-2,2,6,6-tetramethylbicyclo[3.1.0] hexan (H) (M = 210,41 g/mol)

Zu einer Lösung von 87,0 g (0,417 mol) 1-Aza-4-(n-butylimino)-2,2,6,6-tetramethylbicyclo[3.1.0]hexan (F) in 290 ml Methanol werden 15,8 g (0,418 mol) NaBH₄ in kleinen Portionen unter Kühlung bei 16 °C zugegeben. Nach 120 min. erwärmt sich der Ansatz auf 38 °C ohne äußere Heizung (leichte Gasentwicklung). Er wird anschließend gekühlt und bei 25 °C weitere 240 min. gerührt. Das Lösungsmittel wird bei 50 mbar / 50 °C entfernt. Der Rückstand wird mit 200 ml Toluol versetzt und zweimal mit je 100 ml 20 %iger NaCl-Lösung ausgeschüttelt. Die organische Phase wird mit Natriumsulfat getrocknet und von diesem abfiltriert. Das Produkt wird im Vakuum destilliert. Bei einer Abgangstemperatur von 93 °C / 0,04 mbar wird das Produkt als farbloses, dünnflüssiges Öl isoliert (69,6 g; 0,331 mol; 79%).

### 4.2. Herstellung von Verbindung J (M = 390,74 g/mol)

Eine Lösung von 69,0g (0,178 mol) Verbindung G in 240 ml Methanol wird bei 12 °C unter Kühlung portionsweise mit 7,4 g (0,196 mol) NaBH₄ versetzt. Die Mischung wird 10 h bei 67 °C gerührt. Nach dem Entfernen des Lösungsmittels bei 25 mbar und 50 °C wird der Rückstand mit 200 ml Methyl-tert.butylether versetzt und dreimal mit je 100 ml einer 10 %igen NaCl-Lösung ausgeschüttelt. Die organische Phase wird mit Natriumsulfat getrocknet, von diesem abfiltriert und bei 25 mbar / 50 °C vom Lösungsmittel befreit. Die Rohausbeute (61,0 g; 88%; braunes Öl) wird im Vakuum destilliert. Bei einer Abgangstemperatur von 174 °C / 0,015 mbar wird das Produkt als goldgelbes, hochviskoses Öl isoliert (45,0 g; 65%).

### 4.3. Herstellung von Verbindung K (M = 328,53 g/mol)

Unter starkem Rühren werden 13,1 g (0,11 mol) Phenylisocyanat bei 24 °C im Laufe von 5 min. in eine Lösung von 21,0 g (0,1 mol) 1-Aza-4-(n-butylamino)-2,2,6,6-tetramethylbicyclo[3.1.0]hexan (H) in 75 ml Toluol zugetropft. Die Lösung erwärmt sich dabei ohne äußere Heizung auf 68 °C. Die gut gerührte Mischung kühlt sich im Laufe von 30 min. auf 32 °C ab, anschließend wird das Lösungsmittel bei 25 mbar / 50 °C entfernt. Das Rohprodukt (35,5 g; >100%; braunes Öl) wird im Vakuum destilliert. Bei einer Abgangstemperatur von 172 °C / 0,05 mbar wird das Produkt als goldgelbes, hochviskoses Öl isoliert (31,5 g; 0,096 mol; 96%).

### 4.4. Herstellung von Verbindung L (M = 333,58 g/mol)

Unter starkem Rühren werden 13,8 g (0,11 mol) Cyclohexylisocyanat bei 22 °C im Laufe von 5 min. in eine Lösung von 21,0 g (0,10 mol) 1-Aza-4-(n-butylamino)-2,2,6,6-tetramethylbicyclo[3.1.0]hexan (H) in 75 ml Toluol getropft. Die Lösung erwärmt sich dabei ohne äußere Heizung auf 50 °C. Die gut gerührte Mischung kühlt sich im Laufe von 25 h auf 22 °C ab, anschließend wird das Lösungsmittel bei 25 mbar / 50 °C entfernt. Das Rohprodukt (35,8 g; >100%; braunes Öl) wird im Vakuum destilliert. Bei einer Abgangstemperatur von 158 °C / 0,005 mbar wird das Produkt als hellgelbes, hochviskoses Öl isoliert (30,0 g; 0,090 mol; 90%).

### 4.5. Herstellung von Verbindung M (M = 589,04 g/mol)

Unter starkem Rühren werden 8,4 g (0,05 mol) Hexamethylendiisocyanat bei 16 °C im Laufe von 5 min. in eine Lösung von 21,0 g (0,1 mol) 1-Aza-4-(n-butylamino)-2,2,6,6-tetramethylbicyclo[3.1.0]hexan (H) in 75 ml Toluol zugetropft. Die Lösung erwärmt sich dabei ohne äußere Heizung auf 36 °C. Die gut gerührte, farblose Mischung kühlt sich im Laufe von 30 min. auf 32 °C ab, anschließend wird das Lösungsmittel bei 25 mbar / 125 °C entfernt. Das Produkt wird als farbloses, hochviskoses Öl isoliert (28,6 g; 0,049 mol; 98%). ¹H-NMR- und ¹³C-NMR-Spektrum stimmen mit der erwarteten Struktur überein und sind sauber (Carbonylsignal in CDCl₃ bei δ=152,93 ppm).

### 5. Lichtstabilisierende Wirkung in Polypropylenfolien

100 Gewichtsteile unstabilisiertes Polypropylen (®Hostalen PPK) wurden zusammen mit 0,1 Gewichtsteilen Calciumstearat, 0,05 Gewichtsteilen Bis-3,3-bis-(4'-hydroxy-3'-tert.-butyl-phenyl)-butansäure-glykolester (®Hostanox O 3), 0,1 Gewichtsteilen Tris(2,4-di-tert.-butylphenyl)phosphit (®Hostanox PAR 24) und 0,2 Gewichtsteilen des zu prüfenden Stabilisators in einem Schaufelkneter (Hersteller Fa. Brabender) 10 min bei 200°C und 20 U/min geknetet. Aus dieser Mischung wurde bei 190°C eine 100 mm starke Folie gepreßt und die auf diese Weise erhaltenen Prüfkörper in einem Schnellbewitterungsgerät (®Xenotest 450) belichtet. Als Kriterium der Stabilität der Folie wurde die Veränderung des Carbonylindexes innerhalb dieser Zeitspanne herangezogen. Der Carbonylindex CO wurde hierbei gemäß der Formel CO = E₁₇₂₀/E₂₀₂₀ bestimmt, wobei E die Extinktion bei der jeweiligen Wellenlänge bedeutet. Zu Vergleichszwecken wurde eine Folie unter den gleichen Bedingungen, jedoch ohne den Zusatz eines erfindungsgemäßen Stabilisators getestet. Die Versuchsergebnisse sind in Tabelle 1 zusammengestellt:

**Tabelle 1**

| Veränderung des Carbonylindexes erfindungsgemäß stabilisierter Folien | |
|---|---|
| Stabilisator | Zeit, nach welcher die Steigerung des Carbonylindexes um 1 Einheit erfolgt (in Stunden) |
| kein Stabilisator | 350 h |
| Stabilisator E1* | 1060 h |
| Stabilisator E2** | 1990 h |
| Stabilisator J | 830 h |

| | |
|---|---|
| * Produkt E, mit dem kristallinen Anteil des Produktes III hergestellt | |
| ** Produkt E, mit dem öligen Anteil des Produktes III hergestellt | |

Tabelle 1 unterstreicht die sehr gute Lichtschutzwirkung der erfindungsgemäßen Stabilisatoren in Polypropylen.

## Patentansprüche

1. Neue Stabilisatoren der allgemeinen Formel (I) 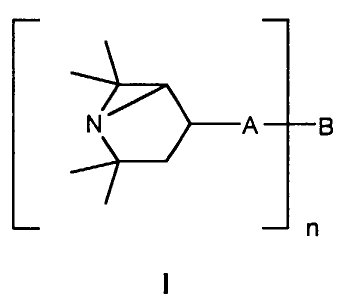 worin
**n** 1 oder 2
**A** -O-C(O)-, -O-C(O)-N(H)-, -N(R¹)-C(O)-, -N(R¹)-C(O)-N(H)-, oder eine direkte Bindung,
**B** falls **A** eine direkte Bindung darstellt,
für **n** = 1 -N(R¹R²) oder -O-R¹,
für **n** = 2 -N(R¹)-,
**B** falls **A** keine direkte Bindung darstellt,
für **n** = 1 C₂-C₁₈-Alkyl, C₃-C₁₀-Cycloalkyl, mit -CN, -NO₂, Amin oder Halogen substituiertes C₆-C₁₈-Aryl oder C₇-C₁₈-Arylalkyl, oder einen heteroaromatischen Rest mit 5-15 C-Atomen,
für **n** = 2 C₂-C₁₈-Alkylen, mit -CN, -NO₂, Amin oder Halogen substituiertes C₆-C₁₈-Arylen oder C₇-C₁₈-Arylalkylen, oder einen heteroaromatischen Rest mit 5-15 C-Atomen,
**R**^{**1**} H, ein mit einem Derivat des Triazins, mit einem Derivat des 1-Aza-2,2,6,6-tetramethyl-bicyclo[3.1.0]hexans oder mit einem Amin substituiertes C₁-C₁₈-Alkyl oder -Alkylen, C₃-C₁₀-Cycloalkyl, mit -CN, -NO₂, Amin oder Halogen substituiertes C₆-C₁₈-Aryl oder C₇-C₁₈-Arylalkyl, oder einen heteroaromatischen Rest mit 5-15 C-Atomen,
**R**^{**2**} H, ein mit einem Derivat des Triazins oder mit einem Amin substituiertes C₁-C₁₈-Alkyl, C₃-C₁₀-Cycloalkyl, mit -CN, -NO₂, Amin oder Halogen substituiertes C₆-C₁₈-Aryl oder C₇-C₁₈-Arylalkyl, oder einen heteroaromatischen Rest mit 5-15 C-Atomen, oder ein Derivat des Triazins
bedeutet.

2. Stabilisatoren gemäß Anspruch 1, worin
**A** -O-C(O)- oder -O-C(O)-N(H)-,
**B** falls **A** keine direkte Bindung darstellt,
für **n** = 1 C₈-C₁₆-Alkyl, C₄-C₈-Cycloalkyl, mit -CN, -NO₂, Amin oder Halogen substituiertes C₆-C₁₀-Aryl oder C₇-C₁₀-Arylalkyl, oder einen heteroaromatischen Rest mit 6-10 C-Atomen,
für **n** = 2 C₄-C₈-Alkylen, mit -CN, -NO₂, Amin oder Halogen substituiertes C₆-C₁₀-Arylen oder C₇-C₁₀-Arylalkylen, oder einen heteroaromatischen Rest mit 6-10 C-Atomen,
**R**^{**1**} H, ein mit einem Derivat des Triazins oder mit einem Amin substituiertes C₂-C₈-Alkyl oder -Alkylen, mit -CN, -NO₂, Amin oder Halogen substituiertes C₆-C₁₀ - Aryl oder C₇-C₁₀-Arylalkyl, oder einen heteroaromatischen Rest mit 6-10 C-Atomen,
**R**^{**2**} H, ein mit einem Derivat des Triazins oder mit einem Amin substituiertes C₂-C₈-Alkyl, C₃-C₁₀-Cycloalkyl, mit -CN, -NO₂, Amin oder Halogen substituiertes C₆-C₁₀-Aryl oder C₇-C₁₀-Arylalkyl oder einen heteroaromatischen Rest mit 6-10 C-Atomen, oder ein Derivat des Triazins der Formel T₁ oder T₂ 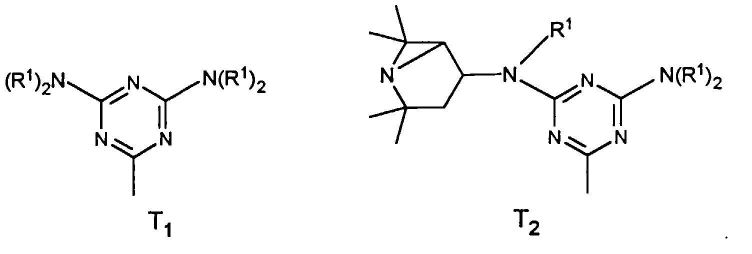
bedeutet.

3. Verfahren zur Herstellung der Verbindungen der Formel (I), dadurch gekennzeichnet, daß in einem ersten Schritt 1-Aza-4-oxo-2,2,6,6-tetramethylbicyclo[3.1.0]hexan (II) zu Alkoholen oder Aminen der Formel (III) oder (V) umgesetzt wird, 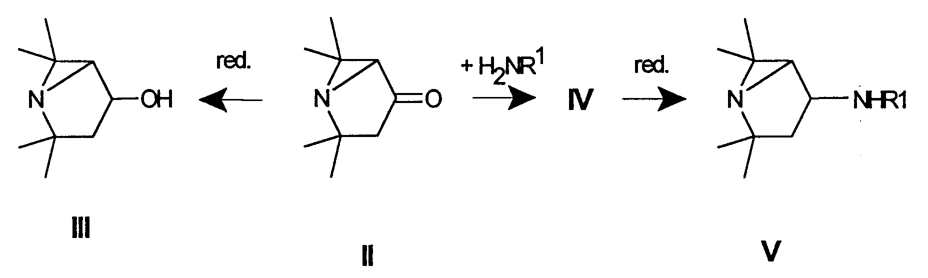 und daß in einem zweiten Schritt diese Reaktionsprodukte mit gegebenenfalls weiter substituierten mono- oder difunktionellen Isocyanaten, Estern, Säurehalogeniden, Säureanhydriden, Alkylhalogeniden, halogensubstituierten Triazinen oder anderen Verbindungen, die mit Alkoholen, primären oder sekundären Aminen reagieren, umgesetzt werden.

4. Verfahren gemäß Anspruch 3, dadurch gekennzeichnet, daß die Umsetzung in einem protischen oder aprotischen, organischen Lösemittel, vorzugsweise einem Kohlenwasserstoff, insbesondere aromatischen Kohlenwasserstoffen, erfolgt.

5. Verfahren gemäß Anspruch 4, dadurch gekennzeichnet, daß es sich bei dem organischen Lösemittel um Toluol, Xylol oder Gemische hieraus oder um Tetrahydrofuran handelt.

6. Verfahren gemäß Anspruch 3, dadurch gekennzeichnet, daß eine der Reaktionskomponenten im Überschuß als Lösemittel verwendet wird.

7. Verwendung der Verbindungen der Formel (I) gemäß Anspruch 1 zum Stabilisieren von organischem Material, wobei es sich hier um Vorprodukte für Kunststoffe, Lacke, Anstrichmittel und Öle, vorzugsweise jedoch um Kunststoffe Lacke, Anstrichmittel oder Öle selbst handelt.

8. Verfahren zum Stabilisieren von polymeren Material gegen den Abbau durch Licht, Strahlung, Wärme und Sauerstoff, dadurch gekennzeichnet, daß man dem zu stabilisierenden Material eine Verbindung der Formel (I) nach Anspruch 1 in einer Konzentration von 0,001 - 5 Gew.%, bevorzugt von 0,1 - 2,0 Gew.% zusetzt.

9. Verfahren gemäß Anspruch 8, dadurch gekennzeichnet, daß man als zusätzliche Additive Antioxidantien, Lichtstabilisatoren, Metalldesaktivatoren, Antistatika, flammhemmende Mittel, Pigmente oder Füllstoffe einsetzt.

10. Stabilisiertes organisches Material, welches mindestens eine der Verbindungen der allgemeinen Formel (I) gemäß Anspruch 1 enthält.
